# EUROPÄISCHE PATENTSCHRIFT

(11) **EP 3 016 925 B1**
(45) Veröffentlichungstag und Bekanntmachung des Hinweises auf die Patenterteilung: **12.08.2020**
(21) Anmeldenummer: 14738436.6
(22) Anmeldetag: 25.06.2014
(51) Int. Cl.: C07C 29/88, C07C 29/09

(54) **VERFAHREN ZUR HERSTELLUNG VON 3-HEPTANOL AUS EINER MISCHUNG ENTHALTEND 2-EHTHYLHEXANAL UND 3-HEPTYLFORMIAT**
PROCESS FOR THE PREPARATION OF 3-HEPTANOL FROM A MIXTURE CONTAINING 2-EHTHYLHEXANAL AND 3-HEPTYL FORMATE
PROCÉDÉ DE PRODUCTION DE 3-HEPTANOL À PARTIR D'UN MÉLANGE CONTENANT UN 2-ETHYLHEXANAL ET UN 3-HEPTYLFORMIATE

(30) Priorität: 02.07.2013 EP 13174705; 02.07.2013 US 201361841942 P
(43) Veröffentlichungstag der Anmeldung: 11.05.2016
(73) Patentinhaber: BASF SE, 67056 Ludwigshafen am Rhein (DE)
(72) Erfinder: TELES, Joaquim, Henrique, 67165 Waldsee (DE); SIMON, Joachim, 67063 Ludwigshafen (DE); DEHN, Martine, 67061 Ludwigshafen (DE); DANZ, Manuel, 68723 Plankstadt (DE); DEHN, Richard, 67061 Ludwigshafen (DE)
(74) Vertreter: BASF IP Association
(86) Internationale Anmeldenummer: PCT/EP2014/063397
(87) Internationale Veröffentlichungsnummer: WO 2015/000762

(56) Entgegenhaltungen:
- DE-A1- 2 139 692

## Beschreibung

Gegenstand der vorliegenden Erfindung ist ein Verfahren zur Herstellung von 3-Heptanol oder einer Mischung enthaltend 3-Heptanol umfassend den folgenden Schritt: (i) Zugabe einer wässrigen Lösung (A) enthaltend ein oder mehrere Alkalimetallhydroxid(e) zu einer Mischung (B) mindestens enthaltend 2-Ethylhexanal und 3-Heptylformiat, dadurch gekennzeichnet, dass die Konzentration des Alkalimetallhydroxids / der Alkalimetallhydroxide in der wässrigen Lösung (A) mindestens 40 Gew.-% beträgt.

Ein weiterer Aspekt der vorliegenden Erfindung betrifft eine Mischung enthaltend 3-Heptanol, wobei die Mischung vorzugsweise herstellbar oder hergestellt ist durch das erfindungsgemäße Verfahren, und wobei die Mischung 2-Ethylhexanal und 3-Heptylformiat in einer Gesamtkonzentration von < 5 Gew.-%, bevorzugt < 2 Gew.-% bezogen auf die Mischung enthält.

3-Heptanol kann industriell z.B. als Nebenproduckt aus dem Leichtsiederstrom bei der Herstellung von 2-Ethylhexansäure erhalten werden. Der besagte Leichtsiederstrom fällt bei der Destillation des Reaktionsaustrags nach erfolgter Oxidation von 2-Ethylhexanal zu 2-Ethylhexansäure an und enthält i. d. R. etwa 50-60 Gew.-% 3-Heptylformiat, 5-10 Gew.-% 3-Heptanol, 10-15 Gew.-% Heptanone (3-/4-Heptanon), 10-20 Gew.-% 2-Ethylhexanal sowie 2-5 Gew.-% 2-Ethylhexansäure.

3-Heptanol kann in dem Leichtsiederstrom durch basische Hydrolyse von 3-Heptylformiat angereichert werden. Allerdings ist die destillative Abtrennung des erhaltenen 3-Heptanols aus dem Leichtsiederstrom insbesondere wegen des noch im Leichtsiederstrom enthaltenen 2-Ethylhexanal nicht mit vertretbarem Aufwand möglich.

DE 2 139 692 beschreibt ein Verfahren zur Gewinnung von 3-Heptanol aus dem oben beschriebenen Leichtsiederstrom der 2-Ethylhexansäure-Herstellung. Es handelt sich um ein zweistufiges Verfahren, welches diskontinuierlich oder kontinuierlich durchgeführt werden kann. Im ersten Schritt erfolgt die vollständige oder teilweise Entfernung der im Gemisch enthaltenen Säure mittels Wasser oder verdünnter Alkalilauge. Anschließend erfolgt Hydrierung in Gegenwart eines Hydrierkatalysators.

Mit diesem zweistufigen Verfahren werden nach abschließender Destillation Ausbeuten von ca. 80% an 3-Heptanol bezogen auf das im Einsatzmaterial enthaltene 3-Heptylformiat, 3-Heptanol und 3-Heptanon erhalten. Die kontinuierliche Hydrierung wird mit einer Ausbeute von 78% beschrieben.

Nachteilig bei dem in DE 2 139 692 beschriebenen Verfahren sind die benötigten hohen Wasserstoffdrücke von bis zu 400 bar, wodurch die Hydrierung nur in speziellen Höchstdruckreaktoren durchgeführt werden kann. Damit sind höhere Investitionskosten beim Bau der Anlage und erhöhte Fertigungsausgaben durch die zweistufige Verfahrensführung verbunden.

Der vorliegenden Erfindung liegt daher die Aufgabe zugrunde, ein Verfahren zur Herstellung von 3-Heptanol aus dem Leichtsiederstrom der 2-Ethylhexansäure-Produktion bereitzustellen, welches die zuvor beschriebenen Nachteile vermeidet. Insbesondere ist es Aufgabe der vorliegenden Erfindung, ein Verfahren zur Herstellung von 3-Heptanol aus dem Leichtsiederstrom der 2-Ethylhexansäure-Produktion bereitzustellen, bei dem die Ausbeute an 3-Heptanol bezogen auf das Einsatzmaterial gesteigert werden kann.

Es wurde überraschenderweise gefunden, dass die Entfernung des 2-Ethylhexanals aus dem Reaktionsgemisch durch direkte Umsetzung mit Alkalilauge bei Normaldruck oder leichtem Überdruck unter gleichzeitiger Hydrolyse des 3-Heptylformiats zum 3-Heptanol gelingt, wenn die Konzentration der zugesetzen Lauge ≥ 40 Gew.-% beträgt.

Gegenstand der vorliegenden Erfindung ist daher ein Verfahren zur Herstellung von 3-Heptanol oder einer Mischung enthaltend 3-Heptanol umfassend den folgenden Schritt (i): Zugabe einer wässrigen Lösung (A) enthaltend ein oder mehrere Alkalimetallhydroxid(e) zu einer Mischung (B) mindestens enthaltend 2-Ethylhexanal und 3-Heptylformiat dadurch gekennzeichnet, dass die Konzentration des Alkalimetallhydroxids / der Alkalimetallhydroxide in der wässrigen Lösung (A) mindestens 40 Gew.-% beträgt.

In den in DE 2 139 692 aufgeführten Beispielen wird der Leichtsiederstrom mit Wasser und verdünnter Natronlauge bei 30 °C behandelt, um die enthaltenen Carbonsäuren zu entfernen. Die hohe Esterzahl und die geringe OH-Zahl der nach der anschließenden Phasentrennung erhaltenen Ölphase zeigen eindeutig, dass unter diesen Bedingungen keine bzw. nur eine geringfügige Hydrolyse des Formiats stattgefunden hat. Die Umsetzung des Formiats erfolgt demnach während der Hydrierung, wobei zugleich die im Gemisch enthaltenen Ketone und Aldehyde zu den entsprechenden sekundären bzw. primären Alkoholen umgesetzt werden.

Es wurde überraschenderweise gefunden, dass unter den Reaktionsbedingungen des erfindungsgemäßen Verfahrens, möglicherweise aufgrund des starken sterischen Anspruchs der Ethylgruppe in alpha-Stellung zur Carbonylgruppe, keine Homoaldolkondensation des 2-Ethylhexanals stattfindet. Vielmehr geht ein Teil des Ethylhexanals eine Aldolkondensation mit dem in der Mischung ebenfalls enthaltenen 3-/4-Heptanon ein. Im Reaktionsaustrag wird zudem 2-Ethylhexanol nachgewiesen, was auf eine Disproportionierung des 2-Ethlyhexanals hindeutet. Sowohl das Aldolkondensationsprodukt als auch das 2-Ethylhexanol unterscheiden sich im Siedeverhalten deutlich von 3-Heptanol. D. h., die im Reaktionsaustrag verbleibenden Nebenkomponenten sowie die aus dem 2-Ethylhexanal entstehenden Verbindungen können anschließend problemlos destillativ getrennt werden.

Die erfindungsgemäß einzusetzende Mischung (B) enthaltend 2-Ethylhexanal und 3-Heptylformiat kann weiterhin noch 2-Ethylhexansäure und/oder 3-Heptanol und/oder 3/4-Heptanon enthalten.

In einer bevorzugten Ausgestaltung des erfindungsgemäßen Verfahrens besteht die Mischung (B) aus folgenden Bestandteilen:

| | |
|---|---|
| 2-Ethylhexanal: | 10-20 Gew.-% |
| 3-Heptylformiat: | 50-60 Gew.-% |
| 3-Heptanol: | 5-10 Gew.-% |
| 3/4-Heptanon: | 8-15 Gew.-% |
| 2-Ethylhexansäure: | 2-5 Gew.-% |
| Wasser: | < 1 Gew.-% |

Erfindungsgemäß kann als Alkalimetallhydroxid Lithiumhydroxid, Natriumhydroxid, Kaliumhydroxid, Cäsiumhydroxid oder eine Kombination zweier oder mehrerer dieser Hydroxide eingesetzt werden. In einer vorteilhaften Ausgestaltung des erfindungsgemäßen Verfahrens ist das Alkalimetallhydroxid / sind die Alkalimetallhydroxide ausgewählt aus der Gruppe bestehend aus Kaliumhydroxid und Natriumhydroxid sowie Kombinationen derselben.

Die Konzentration des Alkalimetallhydroxids / der Alkalimetallhydroxide in der wässrigen Lösung (A) beträgt mindestens 40 Gew.-%. In einer vorteilhaften Ausgestaltung des erfindungsgemäßen Verfahrens beträgt die Konzentration des Alkalimetallhydroxids / der Alkalimetallhydroxide in der wässrigen Lösung (A) mindestens 45 Gew.-%.

In einer weiteren vorteilhaften Ausgestaltung des erfindungsgemäßen Verfahrens beträgt die Molmenge des Alkalimetallhydroxids / der Alkalimetallhydroxide mindestens das 1,5-fache, vorzugsweise mindestens das 2-fache, der Summe der Molmengen von 3-Heptylformiat und 2-Ethylhexanal in der Mischung (B).

Es ist erfindungsgemäß bevorzugt, dass die Zugabe der wässrigen Lösung (A) zu der Mischung (B) über einen Zeitraum von mindestens 3 Stunden erfolgt.

Außerdem ist es erfindungsgemäß bevorzugt, dass nach erfolgter Zugabe der wässrigen Lösung (A) zu der Mischung (B) das erhaltene Reaktionsgemisch (C) über einen Zeitraum von mindestens 6 Stunden, vorzugsweise über einen Zeitraum von mindestens 9 Stunden, besonders bevorzugt über einen Zeitraum von mindestens 12 Stunden, gerührt wird.

Weiterhin ist es erfindungsgemäß bevorzugt, dass die Zugabe der wässrigen Lösung (A) zu der Mischung (B) bei einer Temperatur von mindestens 50 °C, vorzugsweise bei einer Temperatur von mindestens 100 °C, besonders bevorzugt bei einer Temperatur von mindestens 120 °C, erfolgt.

Darüber hinaus ist es erfindungsgemäß bevorzugt, dass die Zugabe der wässrigen Lösung (A) zu der Mischung (B) bei einem Druck von höchstens 3 bar, vorzugsweise bei einem Druck von höchstens 1 bar, erfolgt.

Nach erfolgter Zugabe der wässrigen Lösung (A) zu der Mischung (B) kann das erhaltene Reaktionsgemisch in einem weiteren Reaktionsschritt (ii) mit Wasser versetzt und die sich bildenden Phasen getrennt werden.

Die erhaltene organische Phase kann in einem weiteren Reaktionsschritt (iii) mit Wasser gewaschen und optional anschließend getrocknet werden, beispielsweise mit Hilfe von wasserfreiem Natriumsulfat oder anderen geeigneten Trocknungsmitteln, oder durch Strippen/Destillation.

Durch das erfindungsgemäße Verfahren können die im Reaktionsaustrag der Oxidation von 2-Ethylhexanal zu 2-Ethylhexansäure verbleibenden Nebenkomponenten sowie die aus dem 2-Ethylhexanal entstehenden Verbindungen problemlos destillativ getrennt werden. In einer vorteilhaften Ausgestaltung des erfindungsgemäßen Verfahrens wird die gewaschene und getrocknete organische Phase zur Auftrennung in ihre Bestandteile in einem weiteren Reaktionsschritt (iv) destilliert.

Gegenstand der vorliegenden Erfindung ist weiterhin eine Mischung enthaltend 3-Heptanol, wobei die Mischung vorzugsweise herstellbar oder hergestellt ist durch das erfindungsgemäße Verfahren, und wobei die Mischung 2-Ethylhexanal und 3-Heptylformiat in einer Gesamtkonzentration von < 5 Gew.-%, vorzugsweise < 2 Gew.-% bezogen auf die Mischung enthält.

Die vorliegende Erfindung soll anhand der folgenden Beispiele näher erläutert werden.

### Beispiele 1-12:

Als Einsatzmaterial wird der unbehandelte Leichtsiederstrom aus der Ethylhexansäure-Herstellung verwendet. Die Zusammensetzung dieses Stroms kann dabei innerhalb folgender Grenzen variieren:
3-Heptylformiat: 50-60 Gew.-%,
3-Heptanol: 5-10 Gew.-%,
2-Ethylhexanal: 10-20 Gew.-%,
3/4-Heptanon: 8-15 Gew.-%,
2-Ethylhexansäure: 2-5 Gew.-%,
Wasser: 0,6-0,9 Gew.-%.

Die Säurezahl beträgt 43, die Esterzahl beträgt 249.

Die in Tabelle 1 jeweils angegebene Basen-Menge (Äquivalente) ist bezogen auf die Summe der Molmengen von 3-Heptylformiat, 2-Ethylhexanal und 2-Ethylhexansäure im Einsatzmaterial, die Basen-Konzentration ist bezogen auf die eingesetzte wässrige Lösung.

Umsatz und Ausbeute werden mittels quantitativer GC-Analytik des Rohmaterials bestimmt.

Die Ausbeute an 3-Heptanol bezieht sich auf die Summe aus 3-Heptylformiat und 3-Heptanol im Einsatzmaterial.

### Allgemeine Vorschrift für Versuche bei Normaldruck (Beispiele 1 bis 10):

Der Leichtsiedervorlauf wird in einem 1 L Doppelmantelgefäß vorgelegt und auf die jeweilige Temperatur erhitzt. Anschließend wird die Base als wässrige Lösung über 3 h zugetropft. Nach Beendigung des Zutropfens wird für die in der Tabelle angegebene Zeit bei der angegebenen Temperatur nachgerührt ("Reaktionszeit").

Anschließend wird das Gemisch zur Auflösung der ausgefallenen Salze mit Wasser versetzt und die Phasen bei 50°C getrennt. Umsatz und Ausbeute werden mittels quantitativer GC-Analytik der organischen Phase bestimmt.

### Allgemeine Vorschrift für Druckversuche (Beispiele 11 und 12):

Leichtsiedervorlauf und wässrige Alkalilauge werden in einem 300 ml Stahlautoklaven vorgelegt und auf die jeweilige Temperatur erhitzt. Sodann wird für die in der Tabelle angegebene Zeit bei der angegebenen Temperatur nachgerührt ("Reaktionszeit").

Anschließend wird das Gemisch zur Auflösung der ausgefallenen Salze mit Wasser versetzt, und die Phasen werden bei 50°C getrennt. Umsatz und Ausbeute werden mittels quantitativer GC-Analytik der organischen Phase bestimmt.

**Tabelle 1: Übersicht Versuche**

| **Beispiel** | **Base** | **Konzentration** | **Äquiv.** | **Temperatur** | **Druck** | **Reaktionszeit** | **Umsatz 2-Ethylhexa nal** | **Ausbeute 3-Heptanol** |
|---|---|---|---|---|---|---|---|---|
| | | **[%]** | | **[°C]** | **[bar]** | **[h]** | **[%]** | **[%]** |
| **1** | NaOH | 50 | 3 | 50 | 1 | 21 | 93 | 93 |
| **2** | NaOH | 50 | 3 | 80 | 1 | 12 | 97 | 89 |
| **3** | NaOH | 50 | 3 | 100 | 1 | 12 | 99 | 86 |
| **4** | NaOH | 50 | 3 | 120 | 1 | 6 | 99 | 82 |
| **5** | KOH | 50 | 3 | 120 | 1 | 12 | 99 | 83 |
| **6** | NaOH | 50 | 2 | 100 | 1 | 12 | 94 | 88 |
| **7** | NaOH | 50 | 2 | 120 | 1 | 6 | 94 | 83 |
| | | | | | | 12 | 99 | 81 |
| **8** | NaOH | 50 | 1,5 | 120 | 1 | 12 | 99 | 81 |
| **9 Vergleich** | NaOH | 25 | 2 | 115 | 1 | 21 | 48 | n.b. |
| **10** | NaOH | 40 | 3 | 120 | 1 | 9 | 95 | 87 |
| 11 **Vergleich** | NaOH | 30 | 2,5 | 140 | 3 | 12 | 88 | 71 |
| **12** | NaOH | 40 | 2,5 | 140 | 3 | 12 | 99 | 66 |

### Beispiel 13:

In einem 8 L Doppelmantelgefäß aus Glas, versehen mit einem mechanischen Rührer, werden 2400 g Vorlauf aus der Ethylhexansäureherstellung (Zusammensetzung: 6,2 Gew.-% 3-Heptanol, 8,4 Gew.-% 3-Heptanon, 1,4 Gew.-% 4-Heptanon, 17,2 Gew.-% 2-Ethylhexanal, 53,9 Gew.-% 3-Heptylformiat, 0,8 Gew.-% 2-Ethylhexansäure, 0,9 Gew.-% Wasser) vorgelegt und bei Umgebungsdruck auf 120°C (Rückfluss) erhitzt (Rührerdrehzahl 300 U/min). Dann werden 2960 g 50%ige wässrige NaOH-Lösung (37 mol, 3 mol/mol) über 60 min zugetropft und das Gemisch anschließend noch weitere 6 h unter Rückfluss erhitzt. Die erhaltene Suspension wird mit 1500 g Wasser versetzt und auf 80°C abgekühlt. Anschließend werden die Phasen getrennt. Die organische Phase wird ein weiteres Mal mit 1500 g Wasser gewaschen und über wasserfreiem, pulverförmigem Natriumsulfat (ca. 15 g) getrocknet.

Nach Entfernung des Natriumsulfats durch Filtration werden 1853 g einer klaren, leicht gelben Flüssigkeit erhalten (Säurezahl 0,5, Esterzahl 4). Der Gehalt an 3-Heptanol wird mittels kalibrierter GC-Analytik auf 50,1 % bestimmt, entsprechend einer Ausbeute von 78 % bezogen auf die Summe aus 3-Heptanol und 3-Heptylformiat im Einsatzmaterial.

150 g des so erhaltenen Materials werden über eine Kolonne mit Rücklaufteiler (Höhe 210 mm, Durchmesser 22 mm), welche mit 3 mm Glasringen befallt ist, bei 20 mbar Kopfdruck destilliert. Es werden 53,4 g 3-Heptanol bei einer Kopftemperatur von 61-63°C erhalten (Reinheit >98%).

## Patentansprüche

1. Verfahren zur Herstellung von 3-Heptanol oder einer Mischung enthaltend 3-Heptanol umfassend den folgenden Schritt:
(i) Zugabe einer wässrigen Lösung (A) enthaltend ein oder mehrere Alkalimetallhydroxid(e) zu einer Mischung (B) mindestens enthaltend 2-Ethylhexanal und 3-Heptylformiat,
**dadurch gekennzeichnet, dass** die Konzentration des Alkalimetallhydroxids / der Alkalimetallhydroxide in der wässrigen Lösung (A) mindestens 40 Gew.-% beträgt.

2. Verfahren nach Anspruch 1, **dadurch gekennzeichnet, dass** die Mischung (B) weiterhin eine, zwei oder sämtliche der Verbindungen ausgewählt aus der Gruppe bestehend aus 2-Ethylhexansäure, 3-Heptanol und 3-Heptanon enthält.

3. Verfahren nach Anspruch 2, **dadurch gekennzeichnet, dass** die Mischung (B) aus folgenden Bestandteilen besteht:
| | |
|---|---|
| 2-Ethylhexanal: | 10-20 Gew.-% |
| 3-Heptylformiat: | 50-60 Gew.-% |
| 3-Heptanol: | 5-10 Gew.-% |
| 3/4-Heptanon: | 8-15 Gew.-% |
| 2-Ethylhexansäure: | 2-5 Gew.-% |
| Wasser: | < 1 Gew.-% |

4. Verfahren nach mindestens einem der Ansprüche 1 bis 3, **dadurch gekennzeichnet, dass** das Alkalimetallhydroxid / die Alkalimetallhydroxide ausgewählt ist / sind aus der Gruppe bestehend aus Kaliumhydroxid und Natriumhydroxid sowie Kombinationen derselben.

5. Verfahren nach mindestens einem der Ansprüche 1 bis 4, **dadurch gekennzeichnet, dass** die Konzentration des Alkalimetallhydroxids /der Alkalimetallhydroxide in der wässrigen Lösung (A) mindestens 45 Gew.-% beträgt.

6. Verfahren nach mindestens einem der Ansprüche 1 bis 5, **dadurch gekennzeichnet, dass** die Molmenge des Alkalimetallhydroxids / der Alkalimetallhydroxide mindestens das 1,5-fache, vorzugsweise mindestens das 2-fache, der Summe der Molmengen von 3-Heptylformiat, 2-Ethylhexanal und 2-Ethylhexansäure in der Mischung (B) beträgt.

7. Verfahren nach mindestens einem der Ansprüche 1 bis 6, **dadurch gekennzeichnet, dass** die Zugabe der wässrigen Lösung (A) zu der Mischung (B) über einen Zeitraum von mindestens 3 Stunden erfolgt.

8. Verfahren nach mindestens einem der Ansprüche 1 bis 7, **dadurch gekennzeichnet, dass** nach erfolgter Zugabe der wässrigen Lösung (A) zu der Mischung (B) das resultierende Gemisch (C) über einen Zeitraum von mindestens 6 Stunden, vorzugsweise über einen Zeitraum von mindestens 9 Stunden, besonders bevorzugt über einen Zeitraum von mindestens 12 Stunden, gerührt wird.

9. Verfahren nach mindestens einem der Ansprüche 1 bis 8, **dadurch gekennzeichnet, dass** die Zugabe der wässrigen Lösung (A) zu der Mischung (B) bei einer Temperatur von mindestens 50 °C, vorzugsweise bei einer Temperatur von mindestens 100 °C, besonders bevorzugt bei einer Temperatur von mindestens 120 °C, erfolgt.

10. Verfahren nach mindestens einem der Ansprüche 1 bis 9, **dadurch gekennzeichnet, dass** die Zugabe der wässrigen Lösung (A) zu der Mischung (B) bei einem Druck von höchstens 3 bar, vorzugsweise bei einem Druck von höchstens 1 bar, erfolgt.

11. Verfahren nach mindestens einem der Ansprüche 1 bis 10, **dadurch gekennzeichnet, dass** nach erfolgter Zugabe der wässrigen Lösung (A) zu der Mischung (B) das resultierende Gemisch (C) in einem weiteren Reaktionsschritt (ii) mit Wasser versetzt wird und die sich bildenden Phasen getrennt werden.

12. Verfahren nach Anspruch 11, **dadurch gekennzeichnet, dass** in einem weiteren Reaktionsschritt (iii) die erhaltene organische Phase mit Wasser gewaschen und optional anschließend getrocknet wird.

13. Verfahren nach Anspruch 12, **dadurch gekennzeichnet, dass** in einem weiteren Reaktionsschritt (iv) die gewaschene und gegebenenfalls getrocknete organische Phase zur Auftrennung in ihre Bestandteile destilliert wird.

## Claims

1. A process for the preparation of 3-heptanol or of a mixture comprising 3-heptanol, comprising the following step:
(i) addition of an aqueous solution (A) comprising one or more alkali metal hydroxide (s) to a mixture (B) at least comprising 2-ethylhexanal and 3-heptyl formate,
wherein the concentration of the alkali metal hydroxide/alkali metal hydroxides in the aqueous solution (A) is at least 40% by weight.

2. The process according to claim 1, wherein the mixture (B) furthermore comprises one, two or all of the compounds selected from the group consisting of 2-ethylhexanoic acid, 3-heptanol and 3-heptanone.

3. The process according to claim 2, wherein the mixture (B) consists of the following constituents:
| | |
|---|---|
| 2-ethylhexanal: | 10-20% by weight |
| 3-heptyl formate: | 50-60% by weight |
| 3-heptanol: | 5-10% by weight |
| 3/4-heptanone: | 8-15% by weight |
| 2-ethylhexanoic acid: | 2-5% by weight |
| water: | < 1% by weight |

4. The process according to at least one of claims 1 to 3, wherein the alkali metal hydroxide/alkali metal hydroxides is/are selected from the group consisting of potassium hydroxide and sodium hydroxide, and combinations thereof.

5. The process according to at least one of claims 1 to 4, wherein the concentration of the alkali metal hydroxide/alkali metal hydroxides in the aqueous solution (A) is at least 45% by weight.

6. The process according to at least one of claims 1 to 5, wherein the molar amount of the alkali metal hydroxide/alkali metal hydroxides is at least 1.5 times, preferably at least 2 times, the sum of the molar amounts of 3-heptyl formate, 2-ethylhexanal and 2-ethylhexanoic acid in the mixture (B).

7. The process according to at least one of claims 1 to 6, wherein the addition of the aqueous solution (A) to the mixture (B) takes place over a period of at least 3 hours.

8. The process according to at least one of claims 1 to 7, wherein, after the addition of the aqueous solution (A) to the mixture (B) has taken place, the resulting mixture (C) is stirred for a period of at least 6 hours, preferably for a period of at least 9 hours, particularly preferably for a period of at least 12 hours.

9. The process according to at least one of claims 1 to 8, wherein the addition of the aqueous solution (A) to the mixture (B) takes place at a temperature of at least 50°C, preferably at a temperature of at least 100°C, particularly preferably at a temperature of at least 120°C.

10. The process according to at least one of claims 1 to 9, wherein the addition of the aqueous solution (A) to the mixture (B) takes place at a pressure of at most 3 bar, preferably at a pressure of at most 1 bar.

11. The process according to at least one of claims 1 to 10, wherein, after the addition of the aqueous solution (A) to the mixture (B) has taken place, the resulting mixture (C) is admixed in a further reaction step (ii) with water and the phases that are formed are separated.

12. The process according to claim 11, wherein, in a further reaction step (iii), the resulting organic phase is washed with water and is optionally then dried.

13. The process according to claim 12, wherein, in a further reaction step (iv), the washed and optionally dried organic phase is distilled for separation into its constituents.

## Revendications

1. Procédé pour la préparation de 3-heptanol ou d'un mélange contenant du 3-heptanol comprenant l'étape suivante :
(i) addition d'une solution aqueuse (A) contenant un ou plusieurs hydroxyde(s) de métal alcalin à un mélange (B) contenant au moins du 2-éthylhexanal et du formiate de 3-heptyle,
**caractérisé en ce que** la concentration de l'hydroxyde/des hydroxydes de métal alcalin dans la solution aqueuse (A) est d'au moins 40% en poids.

2. Procédé selon la revendication 1, **caractérisé en ce que** le mélange (B) contient en outre un, deux ou tous les composés choisi(s) dans le groupe constitué par l'acide 2-éthylhexanoïque, le 3-heptanol et la 3-heptanone.

3. Procédé selon la revendication 2, **caractérisé en ce que** le mélange (B) est constitué des ingrédients suivants :
| | |
|---|---|
| 2-éthylhexanal : | 10 à 20% en poids |
| formiate de 3-heptyle : | 50 à 60% en poids |
| 3-heptanol : | 5 à 10% en poids |
| 3/4-heptanone : | 8 à 15% en poids |
| acide 2-éthylhexanoïque : | 2 à 5% en poids |
| eau : | < 1% en poids. |

4. Procédé selon au moins l'une quelconque des revendications 1 à 3, **caractérisé en ce que** l'hydroxyde/les hydroxydes de métal alcalin est/sont choisi (s) dans le groupe constitué par l'hydroxyde de potassium et l'hydroxyde de sodium ainsi que des combinaisons de ceux-ci.

5. Procédé selon au moins l'une quelconque des revendications 1 à 4, **caractérisé en ce que** la concentration de l'hydroxyde/des hydroxydes de métal alcalin dans la solution aqueuse (A) est d'au moins 45% en poids.

6. Procédé selon au moins l'une quelconque des revendications 1 à 5, **caractérisé en ce que** la quantité molaire de l'hydroxyde/des hydroxydes de métal alcalin est d'au moins 1,5 fois, de préférence d'au moins 2 fois la somme des quantités molaires de formiate de 3-heptyle, de 2-éthylhexanal et d'acide 2-éthylhexanoïque dans le mélange (B).

7. Procédé selon au moins l'une quelconque des revendications 1 à 6, **caractérisé en ce que** l'addition de la solution aqueuse (A) au mélange (B) a lieu pendant une période de temps d'au moins 3 heures.

8. Procédé selon au moins l'une quelconque des revendications 1 à 7, **caractérisé en ce qu'**après que l'addition de la solution aqueuse (A) au mélange (B) est réalisée, le mélange résultant (C) est agité pendant une période de temps d'au moins 6 heures, de préférence pendant une période de temps d'au moins 9 heures, particulièrement préférablement pendant une période de temps d'au moins 12 heures.

9. Procédé selon au moins l'une quelconque des revendications 1 à 8, **caractérisé en ce que** l'addition de la solution aqueuse (A) au mélange (B) a lieu à une température d'au moins 50°C, de préférence à une température d'au moins 100°C, particulièrement préférablement à une température d'au moins 120°C.

10. Procédé selon au moins l'une quelconque des revendications 1 à 9, **caractérisé en ce que** l'addition de la solution aqueuse (A) au mélange (B) a lieu à une pression d'au plus 3 bars, de préférence à une pression d'au plus 1 bar.

11. Procédé selon au moins l'une quelconque des revendications 1 à 10, **caractérisé en ce qu'**après que l'addition de la solution aqueuse (A) au mélange (B) est réalisée, le mélange résultant (C) est mélangé avec de l'eau dans une autre étape de réaction (ii) et les phases qui se forment sont séparées.

12. Procédé selon la revendication 11, **caractérisé en ce que**, dans une autre étape de réaction (iii), la phase organique obtenue est lavée avec de l'eau et est éventuellement ensuite séchée.

13. Procédé selon la revendication 12, **caractérisé en ce que** dans une autre étape de réaction (iv) la phase organique lavée et éventuellement séchée est distillée pour la séparation en ses ingrédients.
